(19)

Europäisches
Patentamt
European
Patent Office
Office européen
des brevets

(11) **EP 3 903 926 A1**

(12) **EUROPEAN PATENT APPLICATION**

(43) Date of publication:
**03.11.2021 Bulletin 2021/44**

(21) Application number: **20172354.1**

(22) Date of filing: **30.04.2020**

(51) Int Cl.:
**B01J 21/06** (2006.01)          **B01J 21/08** (2006.01)
**B01J 23/755** (2006.01)          **C07C 209/32** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **BASF SE**
**67056 Ludwigshafen am Rhein (DE)**

(72) Inventors:
• **HEIDEMANN, Thomas**
**67056 Ludwigshafen am Rhein (DE)**
• **SCHWABAUER, Inna**
**67056 Ludwigshafen am Rhein (DE)**

• **WUCHER, Barbara**
**67056 Ludwigshafen am Rhein (DE)**
• **SCHROETER, Marie Katrin**
**67056 Ludwigshafen am Rhein (DE)**
• **OEZKOZANOGLU, Claudia**
**67056 Ludwigshafen am Rhein (DE)**
• **FRIKO, Michael**
**67056 Ludwigshafen am Rhein (DE)**
• **BRANDTS, Jim**
**3454 PK de Meern (NL)**

(74) Representative: **Altmann Stößel Dick**
**Patentanwälte PartG mbB**
**Isartorplatz 1**
**80331 München (DE)**

(54) **CATALYTIC MATERIAL COMPRISING NI SUPPORTED ON AN OXIDIC SUPPORT COMPRISING ZR AND SI**

(57)     A catalytic material comprising Ni supported on an oxidic support comprising Zr in oxidic form and Si in oxidic form, wherein the catalytic material comprises equal to or greater than 65 weight-% of Ni, calculated as elemental Ni, wherein the catalytic material exhibits a Ni:Zr atomic ratio in the range of from 8.5 to 50.0.

EP 3 903 926 A1

**Description**

TECHNICAL FIELD

The present invention relates to a catalytic material comprising Ni on a support which comprises Si and Zr, both in oxidic form, and a process for preparation thereof. In addition thereto, the present invention relates to a use of the inventive catalytic material as a catalyst or catalyst component, especially in a hydrogenation reaction.

INTRODUCTION

The catalytic hydrogenation of nitro group-containing compounds is known. Generally, a hydrogenation reaction is carried out either in a fixed-bed reactor or in a batch reactor. On an industrial scale, it is most usual to carry out hydrogenation reactions in the liquid phase with a suspended catalyst, the processes differing with regard to the reaction temperature, the pressure, the catalyst, the solvents and the way in which the reaction is carried out.

The catalytic materials are typically obtained by precipitation from a solution particularly containing a soluble salt of Ni at pH 7 to 10 by means of a basic compound and are processed by filtration, drying and reduction to give the catalytic material. The catalytic materials thus obtained can be used for a several hydrogenation reactions, but their catalytic activity is often unsatisfactory in many cases, since the catalytic materials still contain other components, in particular Zr and Si in oxidic form.

WO 00/51728 A1 relates to a hydrogenating catalyst and a method for producing the same, wherein the catalyst contains Ni on a support, is stabilized and has Ni crystallite with bimodal Ni crystallite size distribution, a Ni content of 60 to 80 weight-%, based on the total weight of the catalyst and a reduction rate of at least 70 %. The catalyst is suitable for the reduction of nitro-groups of aromatics to the respective amine-group containing aromatics.

WO 00/51727 A1 relates to a catalyst, especially for hydrogenating functional groups of organic compositions in the presence of water. Said catalyst contains Ni on a carrier, is reduced and stabilized and is provided with Ni crystallites with a monomodal Ni Crystallite size distribution, a Ni content of 25 to 60 percentage by weight (in relation to the total weight of the catalyst) and a reduction degree of at least 65 %.

WO 95/24964 A1 relates to nickel-containing hydrogenation catalyst essentially comprising 65 to 80 % Ni calculated as Ni oxide, 10 to 25 % Si, calculated as $SiO_2$, 2 to 10 % Zr, calculated as $ZrO_2$, 0 to 10 % Al, calculated as $Al_2O_3$, with the proviso that the sum of the $SiO_2$ and $Al_2O_3$ contents is at least 15 %, with the percentages by weight in relation to the total mass of the catalyst.

EP 0335222 A1 discloses a catalytic material comprising Ni, $Al_2O_3$, and $ZrO_2$ characterized in that 20 to 90 weight-% Ni, based on the total weight of the catalytic material, and 1 to 30 weight-% $Al_2O_3$ and 0.5 to 20 weight-% $ZrO_2$, based on the total weight of Ni.

DE 1257753 discloses a process for preparation of a nickel- and zirconia-containing catalyst including precipitation of a carbonate and subsequent calcination.

US 2564331 discloses a process for preparation of a nickel-zirconium catalyst by precipitation of carbonates from a nickel- and zirconium-containing solution.

It was an object of the present invention to provide an improved catalytic material, in particular with respect to its catalytic activity. More specifically, it was an object of the present invention to provide a catalytic material exhibiting an improved activity in the hydrogenation of nitro group-containing compounds, in particular a higher selectivity towards desired amine group-containing compounds in a hydrogenation reaction of nitro group-containing compounds. Further, it was an object of the present invention to provide a catalytic material exhibiting an improved longevity and a low selectivity towards by-products, in particular when used as a catalyst or as a catalyst component in a hydrogenation reaction. In addition to that, it was an object of the present invention to provide a process for preparation of such a catalytic material and a process for the hydrogenation of nitro group-containing compounds to amine group-containing compounds. Yet further, it was an object of the present invention to provide a process for preparation of such a catalytic material.

Surprisingly, it has been found that a catalytic material can be provided in accordance with the present invention particularly being characterized in that it has a comparatively high Ni content and a specific atomic ratio of Ni:Zr. Surprisingly, said catalytic material permits for an improved catalytic activity especially in the hydrogenation of nitro group-containing compounds. Also, the catalytic material of the present invention in its oxidic form shows an excellent behavior as concerns its reducibility. Additionally, it was found that the catalytic material of the present invention further shows an improved longevity in particular when used as a catalyst or as a catalyst component in a hydrogenation reaction. Further, a process is provided for preparing a catalytic material in accordance with the present invention.

DETAILED DESCRIPTION

Therefore, the present invention relates to a catalytic material comprising Ni supported on an oxidic support comprising Zr in oxidic form and Si in oxidic form, wherein the catalytic material comprises equal to or greater than 65 weight-% of Ni, calculated as elemental Ni and based on the total weight of the catalytic material, wherein the catalytic material exhibits a Ni:Zr atomic ratio in the range of from 8.5 to 50.0.

It is preferred that the catalytic material exhibits a Ni:Zr atomic ratio in the range of from 8.6 to 45.0, more preferably in the range of from 8.8 to 40.0, more preferably in the range of from 9.0 to 35.0, more preferably in the range of from 9.2 to 30.0, more preferably in the range of from 9.3 to 28.0, more preferably in the range of from 9.5 to 26.0, more preferably in the range of from 14.0 to 19.0.

It is preferred that the catalytic material comprises from 70 to 99 weight-%, more preferably from 75 to 95 weight-%, more preferably from 81 to 90 weight-%, more preferably from 85 to 88 weight-%, of Ni, calculated as elemental Ni, based on the total weight of the catalytic material.

It is preferred that the catalytic material comprises from 1 to 20 weight-%, more preferably from 3 to 18 weight-%, more preferably from 4 to 16 weight-%, more preferably from 5 to 14 weight-%, more preferably from 7 to 12 weight-%, more preferably from 10 to 11 weight-%, of Zr, calculated as $ZrO_2$, based on the total weight of the catalytic material.

It is preferred that the catalytic material exhibits a Ni:Si atomic ratio in the range of from 10:1 to 50:1, more preferably in the range of from 15:1 to 45:1, more preferably in the range of from 20:1 to 40:1, more preferably in the range of from 25:1 to 37:1, more preferably in the range of from 34:1 to 36:1.

It is preferred that the catalytic material comprises from 0.5 to 5.3 weight-%, more preferably from 1.0 to 5.2 weight-%, more preferably from 1.5 to 5.0 weight-%, more preferably from 2.0 to 4.5 weight-%, more preferably from 2.1 to 4.1 weight-%, more preferably from 2.2 to 4.0 weight-%, of Si, calculated as $SiO_2$, based on the total weight of the catalytic material.

It is preferred that the catalytic material exhibits a Zr:Si atomic ratio in the range of from 0.1:1 to 10:1, more preferably in the range of from 0.5:1 to 5.0:1, more preferably in the range of from 1.2:1 to 3.0:1, more preferably in the range of from 1.7:1 to 2.5:1, more preferably in the range of from 1.9:1 to 2.3:1, more preferably in the range of from 2.0:1 to 2.2:1.

It is preferred that from 90 to 100 weight-%, more preferably from 95 to 100 weight-%, more preferably from 99 to 100 weight-%, more preferably from 99.9 to 100 weight-%, of the oxidic support comprising Zr in oxidic form and Si in oxidic form consists of Si, Zr, O, and H.

It is preferred that the Ni is in an oxidation state of 0 or +2.

According to a first alternative, it is preferred that the catalytic material is calcined such that in particular the Ni comprised in the catalytic material is an oxidation state of +2. Thus, it is preferred that the catalytic material is calcined, more preferably in a gas atmosphere comprising one or more of oxygen and nitrogen, more preferably air, more preferably in a gas atmosphere having a temperature of at least 400 °C, more preferably in the range of from 400 to 600 °C, more preferably in the range of from 425 to 475 °C.

According to a second alternative, it is preferred that the Ni comprised in the catalytic material is at least partially in the oxidation state of 0. Thus, a portion of the Ni comprised in the catalytic material is in the oxidation state of 0 and a portion of the Ni comprised in the catalytic material is in the oxidation state of +2. It is preferred that equal to or more than 55 atomic-%, more preferably equal to or more than 60 atomic-%, more preferably equal to or more than 65 atomic-%, more preferably equal to or more than 70 atomic-%, more preferably equal to or more than 75 atomic-%, more preferably equal to or more than 80 atomic-%, more preferably equal to or more than 85 atomic-%, of the Ni are in an oxidation state of 0.

In the case where the Ni comprised in the catalytic material is at least partially in the oxidation state of 0, in particular in the case where equal to or more than 55 atomic-%, more preferably equal to or more than 60 atomic-%, more preferably equal to or more than 65 atomic-%, more preferably equal to or more than 70 atomic-%, more preferably equal to or more than 75 atomic-%, more preferably equal to or more than 80 atomic-%, more preferably equal to or more than 85 atomic-%, of the Ni are in an oxidation state of 0, it is preferred that the catalytic material comprises particles of Ni.

In the case where the catalytic material comprises particles of Ni, it is preferred that the catalytic material exhibits a monomodal particle size distribution of the crystallites of Ni, preferably determined according to Reference Example 4.

In the case where the catalytic material exhibits a monomodal particle size distribution of the crystallites of Ni, it is preferred that the particle size distribution of the crystallites of Ni displays a maximum in the range of from 2 to 20 nm, more preferably in the range of from 3 to 15 nm, more preferably in the range of from 4 to 11 nm, more preferably in the range of from 4.5 to 10.0 nm, more preferably in the range of from 5.0 to 9.0 nm, preferably determined according to Reference Example 4.

Further in the case where the Ni comprised in the catalytic material is at least partially in the oxidation state of 0, in particular in the case where equal to or more than 55 atomic-%, more preferably equal to or more than 60 atomic-%, more preferably equal to or more than 65 atomic-%, more preferably equal to or more than 70 atomic-%, more preferably equal to or more than 75 atomic-%, more preferably equal to or more than 80 atomic-%, more preferably equal to or more than 85 atomic-%, of the Ni are in an oxidation state of 0, it is preferred that the Ni has a metallic surface area in the range of from 40 to 100 $m^2/g$, more preferably in the range of from 50 to 90 $m^2/g$, more preferably in the range of from 70 to 80 $m^2/g$, preferably determined according to Reference Example 3.

It is preferred that the catalytic material exhibits a total pore volume in the range of from 0.1 to 1.00 $cm^3/g$, preferably in the range of from 0.20 to 0.50 $cm^3/g$, more preferably in the range of from 0.25 to 0.45 $cm^3/g$, preferably determined according to Reference Example 2.

It is preferred that the catalytic material comprises from 0 to 2 weight-%, more preferably from 0.001 to 1 weight-%, more preferably from 0.01 to 0.5 weight-%, of Hf, calculated as $HfO_2$ It is particularly preferred that the catalytic material is essentially free of Hf. Further, it is particularly preferred that the catalytic material does not comprise Hf.

It is preferred that the catalytic material comprises from 0 to 1 weight-%, more preferably from 0.001 to 0.5 weight-%, more preferably from 0.01 to 0.1 weight-%, of an alkali metal, calculated as elemental alkali metal. It is particularly preferred that the catalytic material is essentially free of an alkali metal. Further, it is particularly preferred that the catalytic material does not comprise an alkali metal. It is preferred that the alkali metal comprises one or more of Li, Na, and K, more preferably Na.

It is preferred that the catalytic material further comprises an element E selected from the group consisting of Mg, Ca, Zn, B, Fe, Cl, and a mixture of two or more thereof.

In the case where the catalytic material further comprises an element E selected from the group consisting of Mg, Ca, Zn, B, Fe, Cl, and a mixture of two or more thereof, it is preferred that the catalytic material comprises from 0 to 1 weight-%, more preferably from 0.001 to 0.5 weight-%, more preferably from 0.01 to 0.1 weight-%, of the element E, calculated as elemental E, based on the total weight of the catalytic material.

According to a first alternative, it is preferred that from 90 to 100 weight-%, more preferably from 95 to 100 weight-%, more preferably from 99 to 100 weight-%, more preferably from 99.9 to 100 weight-%, of the catalytic material consist of Ni, Si, Zr, O and H.

According to a second alternative, it is preferred that from 90 to 100 weight-%, more preferably from 95 to 100 weight-%, more preferably from 99 to 100 weight-%, more preferably from 99.9 to 100 weight-%, of the catalytic material consist of Ni, Si, Zr, optionally Hf, optionally an alkali metal, optionally an element E, optionally C, O and H.

It is preferred that the catalytic material comprises from 0 to 3 weight-%, more preferably from 0.001 to 1 weight-%, more preferably from 0.01 to 0.5 weight-%, of C, calculated as elemental C. It is particularly preferred that the catalytic material is essentially free of C. Further, it is particularly preferred that the catalytic material does not comprise C.

The catalytic material can be present in any conceivable form. It is preferred that the catalytic material is in the form of a molding.

In the case where the catalytic material is in the form of a molding, it is preferred that the catalytic material further comprises an auxiliary agent selected from the group consisting of graphite, a polysaccharide, a sugar alcohol, a synthetic polymer, and a mixture of two or more thereof, more preferably selected from the group consisting of graphite, a sugar alcohol, a synthetic polymer, cellulose, a modified cellulose, a starch, and a mixture of two or more thererof, more preferably selected from the group consisting of graphite, a sugar alcohol, a synthetic polymer, a microcrystalline cellulose, a cellulose ether, and a mixture of two or more thereof, more preferably selected from the group consisting of graphite, sorbitol, mannitol, polyethylene glycol (PEG), polyvinylpyrrolidone (PVP), hydroxypropyl cellulose (HPC), hydroxypropyl methylcellulose (HPMC), and a mixture of two or more thereof, wherein the auxiliary agent more preferably comprises, more preferably consist of, graphite.

Further in the case where the catalytic material is in the form of a molding, it is preferred that the catalytic material comprises the auxiliary agent in an amount in the range of from 1.0 to 5.0 weight-%, more preferably in the range of from 2.0 to 4.0 weight-%, more preferably in the range of from 2.5 to 3.5 weight-%, based on the total weight of the catalytic material.

Further in the case where the catalytic material is in the form of a molding, it is preferred that the catalytic material is in the form of a tablet, more preferably a tablet having a cylindrical shape or an ellipsoidal shape.

In the case where the catalytic material is in the form of a tablet, it is preferred that the tablet has a cylindrical shape, wherein the diameter is in the range of from 5 to 15 mm, more preferably in the range of from 8 to 12 mm, more preferably in the range of from 9 to 11 mm, and wherein the height is preferably in the range of from 3 to 13 mm, preferably in the range of from 6 to 10 mm, more preferably in the range of from 7 to 9 mm.

Further in the case where the catalytic material is in the form of a tablet, it is preferred that the tablet has a cylindrical shape, and wherein the tablet has a side crushing strength in the range of from 70 to 130 N, more preferably in the range of from 80 to 120 N, preferably determined according to Reference Example 5.

Further, the present invention relates to a process for the preparation of a catalytic material, preferably of a catalytic material according to any one of the embodiments disclosed herein, said process comprising

(a) providing a first aqueous solution comprising a source of Si, a second aqueous solution comprising a source of Ni, a third aqueous solution comprising a precipitation agent, and a fourth aqueous solution comprising Zr;

(b) mixing the first aqueous solution, the second aqueous solution, the third aqueous solution, and the fourth aqueous solution;

(c) heating of the mixture obtained in (b) to a temperature in the range of from 50 to 90 °C, to obtain a precursor of the catalytic material;

(d) calcining of the precursor of the catalytic material obtained in (c) in a gas atmosphere having a temperature in the range of from 300 to 600 °C.

It is preferred that in (a) the first aqueous solution is heated to a temperature in the range of from 60 to 80 °C, more preferably in the range of from 65 to 75 °C.

According to a first alternative, it is preferred that (b) comprises

(b.1) adjusting the pH of the first aqueous solution to be in the range of from 5 to 9;

(b.2) feeding the second aqueous solution, the third aqueous solution, and the fourth aqueous solution into the solution obtained in (b.1), to obtain a reaction mixture, such that the pH of the reaction mixture is in the range of from 6 to 8, more preferably in the range of from 6.5 to 7.5, preferably in the range of from 6.9 to 7.1.

In the case where (b) comprises (b.1) and (b.2), it is preferred that in (b.1) the pH is adjusted to be in the range of from 6.5 to 8.5, more preferably in the range of from 6.7 to 8.0, more preferably in the range of from 6.8 to 7.5, more preferably in the range of from 6.9 to 7.1.

Further in the case where (b) comprises (b.1) and (b.2), it is preferred that in (b.1) the pH is adjusted by feeding a fifth aqueous solution comprising a source of Ni into the first aqueous solution, wherein the fifth aqueous solution more preferably has the same chemical composition as the second aqueous solution, wherein more preferably the fourth aqueous solution comprises a portion of the second aqueous solution.

According to a second alternative, it is preferred that (b) comprises

(b.1') mixing the first aqueous solution and the third aqueous solution, to obtain an alkaline water glass-containing solution;

(b.2') mixing the second aqueous solution and the fourth aqueous solution, to obtain a metal-containing aqueous solution

(b.3') mixing the alkaline water glass-containing solution obtained in (b.1') and the metal-containing aqueous solution, to obtain a reaction mixture, such that the pH of the reaction mixture is in the range of from 7 to 9, more preferably in the range of from 7.5 to 8.5, more preferably in the range of from 7.9 to 8.1.

It is preferred that heating in (c) comprises heating of the mixture to a temperature in the range of from 55 to 90 °C, more preferably in the range of from 65 to 80 °C, more preferably in the range of from 70 to 75 °C.

It is preferred that the source of Si in the one or more of the first aqueous solution and the third aqueous solution comprises one or more of a silicate, more preferably one or more of a metasilicate, an orthosilicate, and a pyrosilicate, more preferably a silicate salt, more preferably a silicate salt selected from the group consisting of alkali metal silicates and mixtures thereof, wherein the alkali metal is preferably selected from the group consisting of Li, Na, K, Rb, Cs, and mixtures of two or more thereof, more preferably from the group consisting of Li, Na, K, and mixtures of two or more thereof, wherein more preferably the alkali metal is Na and/or K, preferably Na, wherein the source of Si preferably comprises, more preferably consists of, a metasilicate, more preferably sodium silicate.

It is preferred that the concentration of the source of Si, calculated as $SiO_2$, in the first aqueous solution is in the range of from 10 to 500 mmol/l, more preferably in the range of from 30 to 250 mmol/l, more preferably in the range of from 50 to 100 mmol/l.

It is preferred that the source of Ni comprises one or more Ni salts, more preferably one or more Ni(II) salts, wherein the anion of the one or more Ni salts is preferably selected from the group consisting of halides, carbonate, hydrogencarbonate, sulfate, hydrogensulfate, hydroxide, nitrate, phosphate, hydogenphosphate, dihydrogenphosphate, acetate, and combinations of two or more thereof,
more preferably from the group consisting of chloride, bromide, fluoride, hydrogencarbonate, hydrogensulfate, nitrate, dihydrogenphosphate, acetate, and combinations of two or more thereof,
more preferably from the group consisting of chloride, fluoride, nitrate, acetate, and combinations of two or more thereof,
wherein more preferably the anion of the one or more Ni salts is chloride and/or nitrate, preferably nitrate,
and wherein more preferably the source of Ni comprise Ni(II) chloride, wherein more preferably the source of Ni is Ni(II) chloride.

It is preferred that the concentration of the source of Ni, calculated as NiO, in the second aqueous solution and/or in the fifth aqueous solution is in the range of from 0.1 to 4 mol/l, more preferably in the range of from 0.4 to 2.5 mol/l, more preferably in the range of from 0.7 to 1.4 mol/l.

It is preferred that the ratio of the total volume of the first aqueous solution to the total volume of the second aqueous solution is in the range of from 1:50 to 1:2, more preferably in the range of from 1:30 to 1:5, more preferably in the range of from 1:10 to 1:6.

It is preferred that the precipitation agent comprises one or more of an inorganic base and an organic base, more preferably an inorganic base, wherein preferably the precipitation agent is selected from the group consisting of hydroxides, carbonates, aluminates, and mixtures of two or more thereof,

more preferably from the group consisting of alkali metal hydroxides, alkali metal carbonates, alkali metal hydrogen carbonates, alkali metal aluminates, and mixtures of two or more thereof, more preferably from the group consisting of alkali metal carbonates, alkali metal hydrogen carbonates, alkali metal aluminates, and mixtures of two or more thereof, wherein the alkali metal is preferably selected from the group consisting of Li, Na, K, Rb, Cs, and mixtures of two or more thereof,

more preferably from the group consisting of Li, Na, K, and mixtures of two or more thereof, wherein more preferably the alkali metal is Na and/or K, preferably Na,

and wherein more preferably the precipitation agent comprises sodium carbonate, sodium hydrogen carbonate, and/or sodium aluminate, preferably sodium carbonate, wherein more preferably the precipitation agent is sodium carbonate and/or sodium hydrogen carbonate, preferably sodium carbonate.

It is preferred that the concentration of the precipitation agent in the third aqueous solution is in the range of from 1.0 to 5.0 mol/l, more preferably in the range of from 1.4 to 3.0 mol/l, more preferably in the range of from 1.8 to 2.4 mol/l.

It is preferred that the ratio of the total volume of the first aqueous solution to the total volume of the third aqueous solution is in the range of from 1:70 to 5:1, more preferably in the range of from 1:15 to 3:1, more preferably in the range of from 1:2 to 2:1.

It is preferred that the concentration of the source of Zr, calculated as $ZrO_2$, in the fourth aqueous solution is in the range of from 10 to 750 mmol/l, more preferably in the range of from 20 to 600 mmol/l, more preferably in the range of from 30 to 300 mmol/l.

It is preferred that the ratio of the total volume of the first aqueous solution to the total volume of the fourth aqueous solution is in the range of from 1:20 to 1:2, more preferably in the range of from 1:10 to 1:5, more preferably in the range of from 1:8 to 1:6.

It is preferred that the gas atmosphere in (d) comprises a temperature in the range of from 400 to 500 °C, more preferably in the range of from 440 to 460 °C.

It is preferred that the process further comprises after (c) and prior to (d) one or more of

(s) separating the precursor of the catalytic material obtained in (d) from the mixture by filtration or centrifugation, more preferably by filtration;
(t) drying the precursor of the catalytic material obtained from (d) or (s), more preferably the precursor of the catalytic material obtained in (s), in a gas atmosphere having a temperature in the range of from 100 to 140 °C, more preferably in the range of from 110 to 130 °C, more preferably in the range of from 115 to 125 °C.

It is preferred that the gas atmosphere in one or more of (d) and (t) comprises one or more of nitrogen and oxygen, more preferably air.

It is preferred that the process further comprises

(e) treating the catalytic material obtained in (d) in a gas stream comprising nitrogen and hydrogen, wherein the catalytic material is heated to a temperature in the range of from 300 to 450 °C, more preferably in the range of from 360 to 430 °C;
(f) optionally treating the catalytic material obtained in (e) with a gas stream comprising oxygen and one or more of nitrogen and carbon dioxide for passivation, wherein the catalytic material is heated to a temperature in the range of from 30 to 80 °C, more preferably in the range of from 35 to 60 °C.

In the case where the process further comprises (e) and optionally (f), it is preferred that the gas stream in (e) comprises hydrogen in an amount in the range of from 1 to 50 volume-%, based on the total volume of the gas stream, and nitrogen in an amount in the range of from 50 to 99 volume-%, based on the total volume of the gas stream.

Further in the case where the process further comprises (e) and optionally (f), it is preferred that in (e) the content of hydrogen in the gas stream is adjusted such that in (e) the temperature of the catalytic material does not exceed 425 °C. It is particularly preferred that in (e) the temperature of the catalytic material does not exceed 385 °C.

In the case where the process further comprises (f), it is preferred that in (f) the content of oxygen in the gas stream is adjusted such that in (f) the temperature of the catalytic material does not exceed 80 °C. It is particularly preferred that in (f) the temperature of the catalytic material does not exceed 35 °C.

It is preferred that the process further comprises

(A) mixing the catalytic material obtained in (d), (e), or (f) and an auxiliary agent, to obtain a mixture;
(B) shaping the mixture obtained from (A), to obtain a catalytic material being in the form of a molding.

In the case where the process further comprises (A) and (B), it is preferred that the auxiliary agent comprises one or more of graphite, a polysaccharide, a sugar alcohol and a synthetic polymer, more preferably one or more of graphite, a sugar alcohol, a synthetic polymer, cellulose, a modified cellulose and a starch, more preferably one or more of graphite, a sugar alcohol, a synthetic polymer, a microcrystalline cellulose, a cellulose ether, more preferably one or more of graphite, sorbitol, mannitol, polyethylene glycol (PEG), polyvinylpyrrolidone (PVP), hydroxypropyl cellulose (HPC) and hydroxypropyl methylcellulose (HPMC), more preferably graphite.

Further in the case where the process further comprises (A) and (B), it is preferred that the mixture prepared in (A) comprises the auxiliary agent in an amount in the range of from 1.0 to 5.0 weight-%, preferably in the range of from 2.0 to 4.0 weight-%, more preferably in the range of from 2.5 to 3.5 weight-%, based on the total weight of the mixture.

Further in the case where the process further comprises (A) and (B), it is preferred that shaping in (B) comprises extruding or tableting the mixture obtained in (A), more preferably tableting the mixture obtained in (A).

Further in the case where the process further comprises (A) and (B), it is preferred that shaping in (B) comprises tableting the mixture to a tablet, more preferably to a tablet having a cylindrical shape or an ellipsoidal shape.

In the case where tableting is performed to obtain a tablet having a cylindrical shape, it is preferred that the diameter of the tablet is in the range of from 5 to 15 mm, more preferably in the range of from 8 to 12 mm, more preferably in the range of from 9 to 11 mm, and wherein the height is preferably in the range of from 3 to 13 mm, preferably in the range of from 6 to 10 mm, more preferably in the range of from 7 to 9 mm.

Further in the case where the process further comprises (A) and (B), it is preferred that the process further comprises

(C) treating the catalytic material being in the form of a molding obtained in (B) for reducing Ni in a gas stream comprising nitrogen and hydrogen, wherein the catalytic material being in the form of a molding is heated to a temperature in the range of from 325 to 425 °C, more preferably in the range of from 350 to 390 °C;
(D) optionally treating the catalytic material being in the form of a molding obtained from (C) with a gas stream comprising oxygen and one or more of nitrogen and carbon dioxide for passivation, wherein the molding is heated to a temperature in the range of from 30 to 80 °C, more preferably in the range of from 35 to 80 °C.

In the case where the process further comprises (C) and (D), it is preferred that the gas stream in (C) comprises hydrogen in an amount in the range of from 1 to 50 volume-%, based on the total volume of the gas stream, and nitrogen in an amount in the range of from 50 to 99 volume-%, based on the total volume of the gas stream.

Further in the case where the process further comprises (C) and (D), it is preferred that in (C) the content of hydrogen in the gas stream is adjusted such that in (C) the temperature of the molding does not exceed 425 °C. It is particularly preferred that the temperature of the molding does not exceed 380 °C.

Further in the case where the process further comprises (C) and (D), it is preferred that in (D) the content of oxygen in the gas stream is adjusted such that in (D) the temperature of the molding does not exceed 80 °C. It is particularly preferred that the temperature of the molding does not exceed 35 °C.

Yet further, the present invention relates to a catalytic material obtainable or obtained by the process of any one of the embodiments disclosed herein, preferably by a process comprising (a), (b), (c), (s), (t), (e), and (f).

Yet further, the present invention relates to a use of the catalytic material according to any one of the embodiments disclosed herein, as a catalyst or catalyst component, preferably for a hydrogenation reaction, in particular in a hydrogenation reaction of one or more of a nitro-group containing compound, a nitrile, an aromatic, and an olefin.

Yet further, the present invention relates to a continuous process for catalytic hydrogenation of a nitro group-containing compound, the process comprising

(I) providing a reactor comprising a reaction zone which comprises the catalytic material according to any one of the embodiments disclosed herein;
(II) passing a reactant stream into the reaction zone obtained from (A), wherein the reactant stream passed into the reaction zone comprises a nitro group-containing compound and hydrogen; subjecting said reactant gas stream to reaction conditions in said reaction zone; and removing a product stream from said reaction zone, said product stream comprising an amine-group containing compound.

With respect to the reactor provided in (I), it is preferred that a loop reactor is used, in particular a loop Venturi reactor. Thus, it is preferred that a reactor is provided in (I) as disclosed in WO 2014/108351 A1. As an alternative, the reactor provided in (I) is in accordance with a reactor disclosed for the process for preparing amines as disclosed in WO 00/35852 A1.

It is preferred that in (I) the catalytic material is present in a fixed-bed and/or in a fluidized bed, more preferably in a fluidized bed.

It is preferred that in (I) the reaction zone comprises a solvent system, wherein the solvent system more preferably comprises one or more of water, and an amine-group containing compound as obtained in (II), more preferably water, more preferably de-ionized water, more preferably water, which is obtained during the hydrogenation reaction (II).

It is preferred that in (II) the reaction conditions comprise a temperature in the range of from 100 to 150 °C, more preferably in the range of from 110 to 140 °C, more preferably in the range of from 120 to 140 °C.

It is preferred that in (II) the reaction conditions comprise a pressure in the range of from 10 to 150 bar(abs), more preferably in the range of from 15 to 50 bar(abs), more preferably in the range of from 20 to 30 bar(abs).

It is preferred that the nitro group containing compound comprises one or more of an aromatic nitro group-containing compound and an aliphatic nitro group-containing compound, wherein the aromatic nitro group-containing compound more preferably comprises one or more of nitrobenzene, 1,3-dinitrobenzene, 2,4-dinitrotoluene, 2,6-dinitrotoluene, 2,4,6-trinitrotoluene, 1,2-dimethyl-3-nitrobenzene, 1,2-dimethyl-4-nitrobenzene, 1,4-dimethyl-2-nitrobenzene, 1,3-dimethyl-2-nitrobenzene, 2,4-dimethyl-1 nitrobenzene, 1,3-dimethyl-5-nitrobenzene, 1-nitronaphthalene, 2-nitronaphthalene, 1,5-dinitronaphthalene und 1,8-dinitronaphthalene, 2-mononitrotoluene, 3- mononitrotoluene, 4-mononitrotoluene, 2-chloro-1,3-dinitrobenzene, 1-chloro-2,4-dinitrobenzene, o-chloronitrobenzene, m-chloronitrobenzene, p-chloronitrobenzene, 1,2-dichloro-4-nitrobenzene, 1,4-dichloro-2- nitrobenzene, 2,4-dichloro-1-nitrobenzene, 1,2-dichloro-3-nitrobenzene, 4-chloro-2-nitrotoluene, 4-chloro-3-nitrotoluene, 2-chloro-4-nitrotoluene, 2-chlor-6-nitrotoluene, o-nitroaniline, m-nitroaniline, and p-nitroaniline, preferably 2,4-dinitrotoluene or a mixture of 2,4- and 2,6-dinitrotoluene, and wherein the aliphatic nitro group-containing compound more preferably comprises one or more of tris(hydroxymethyl)nitromethane, 2-nitro-2-methyl-1,3-propanediol, 2-nitro-2-ethyl-1,3-propanediol, 2-nitro-1-butanol and 2-nitro-2-methyl-1-propanol.

It is preferred that the product stream comprises an amine group-containing compound, more preferably one or more of an aromatic amine group-containing compound and an aliphatic amine group-containing compound, wherein the aromatic amine group-containing compound preferably comprises one or more of aminobenzene, 1,3-diaminobenzene, 2,4-diaminotoluene, 2,6-diaminotoluene, 2,4,6-triaminotoluene, 1,2-dimethyl-3-aminobenzene, 1,2-dimethyl-4-aminobenzene, 1,4-dimethyl-2-aminobenzene, 1,3-dimethyl-2-aminobenzene, 2,4-dimethyl-1-aminobenzene, 1,3-dimethyl-5-aminobenzene, 1-aminonaphthalene, 2-aminonaphthalene, 1,5-diaminonaphthalene und 1,8-diaminonaphthalene, 2-aminotoluene, 3 aminotoluene, 4-aminotoluene, 2-chloro-1,3-diaminobenzene, 1-chloro-2,4-diaminobenzene, o-chloroaminobenzene, m-chloroaminobenzene, p-chloroaminobenzene, 1,2-dichloro-4-aminobenzene, 1,4-dichloro-2-aminobenzene, 2,4-dichloro-1-aminobenzene, 1,2-dichloro-3-aminobenzene, 4-chloro-2-aminotoluene, 4-chloro-3-aminotoluene, 2-chloro-4-aminotoluene, 2-chlor-6-aminotoluene, o-phenylenediamine, m-phenylenediamine, and p-phenylenediamine, preferably 2,4-diaminotoluene, and wherein the aliphatic amine group-containing compound preferably comprises one or more of tris(hydroxymethyl)aminomethane, 2-amino-2-methyl-1,3-propanediol, 2-amino-2-ethyl-1,3-propanediol, 2-amino-1-butanol and 2-amino-2-methyl-1-propanol.

The unit bar(abs) refers to an absolute pressure of $10^5$ Pa and the unit Angstrom refers to a length of $10^{-10}$ m.

The present invention is further illustrated by the following set of embodiments and combinations of embodiments resulting from the dependencies and back-references as indicated. In particular, it is noted that in each instance where a range of embodiments is mentioned, for example in the context of a term such as "any one of embodiments (1) to (4)", every embodiment in this range is meant to be explicitly disclosed for the skilled person, i.e. the wording of this term is to be understood by the skilled person as being synonymous to "any one of embodiments (1), (2), (3), and (4)".

Further, it is explicitly noted that the following set of embodiments is not the set of claims determining the extent of protection, but represents a suitably structured part of the description directed to general and preferred aspects of the present invention.

According to an embodiment (1), the present invention relates to a catalytic material comprising Ni supported on an oxidic support comprising Zr in oxidic form and Si in oxidic form, wherein the catalytic material comprises equal to or greater than 65 weight-% of Ni, calculated as elemental Ni and based on the total weight of the catalytic material, wherein the catalytic material exhibits a Ni:Zr atomic ratio in the range of from 8.5 to 50.0.

A preferred embodiment (2) concretizing embodiment (1) relates to said catalytic material, wherein the catalytic material exhibits a Ni:Zr atomic ratio in the range of from 8.6 to 45.0, more preferably in the range of from 8.8 to 40.0, more preferably in the range of from 9.0 to 35.0, more preferably in the range of from 9.2 to 30.0, more preferably in the range of from 9.3 to 28.0, more preferably in the range of from 9.5 to 26.0, more preferably in the range of from 14.0 to 19.0.

A further preferred embodiment (3) concretizing embodiment (1) or (2) relates to said catalytic material, wherein the catalytic material comprises from 70 to 99 weight-%, more preferably from 75 to 95 weight-%, more preferably from 81 to 90 weight-%, more preferably from 85 to 88 weight-%, of Ni, calculated as elemental Ni, based on the total weight of the catalytic material.

A further preferred embodiment (4) concretizing any one of embodiments (1) to (3) relates to said catalytic material comprising from 1 to 20 weight-%, more preferably from 3 to 18 weight-%, more preferably from 4 to 16 weight-%, more preferably from 5 to 14 weight-%, more preferably from 7 to 12 weight-%, more preferably from 10 to 11 weight-%, of Zr, calculated as $ZrO_2$, based on the total weight of the catalytic material.

A further preferred embodiment (5) concretizing any one of embodiments (1) to (4) relates to said catalytic material exhibiting a Ni:Si atomic ratio in the range of from 10:1 to 50:1, more preferably in the range of from 15:1 to 45:1, more preferably in the range of from 20:1 to 40:1, more preferably in the range of from 25:1 to 37:1, more preferably in the range of from 34:1 to 36:1.

A further preferred embodiment (6) concretizing any one of embodiments (1) to (5) relates to said catalytic material comprising from 0.5 to 5.3 weight-%, more preferably from 1.0 to 5.2 weight-%, more preferably from 1.5 to 5.0 weight-%, more preferably from 2.0 to 4.5 weight-%, more preferably from 2.1 to 4.1 weight-%, more preferably from 2.2 to 4.0 weight-%, of Si, calculated as $SiO_2$, based on the total weight of the catalytic material.

A further preferred embodiment (7) concretizing any one of embodiments (1) to (6) relates to said catalytic material exhibiting a Zr:Si atomic ratio in the range of from 0.1:1 to 10:1, more preferably in the range of from 0.5:1 to 5.0:1, more preferably in the range of from 1.2:1 to 3.0:1, more preferably in the range of from 1.7:1 to 2.5:1, more preferably in the range of from 1.9:1 to 2.3:1, more preferably in the range of from 2.0:1 to 2.2:1.

A further preferred embodiment (8) concretizing any one of embodiments (1) to (7) relates to said catalytic material, wherein from 90 to 100 weight-%, more preferably from 95 to 100 weight-%, more preferably from 99 to 100 weight-%, more preferably from 99.9 to 100 weight-%, of the oxidic support comprising Zr in oxidic form and Si in oxidic form consists of Si, Zr, O, and H.

A further preferred embodiment (9) concretizing any one of embodiments (1) to (8) relates to said catalytic material, wherein the Ni is in an oxidation state of 0 or +2.

A further preferred embodiment (10) concretizing any one of embodiments (1) to (9) relates to said catalytic material, wherein the catalytic material is calcined, more preferably in a gas atmosphere comprising one or more of oxygen and nitrogen, more preferably air, more preferably in a gas atmosphere having a temperature of at least 400 °C, more preferably in the range of from 400 to 600 °C, more preferably in the range of from 425 to 475 °C.

A further preferred embodiment (11) concretizing any one of embodiments (1) to (9) relates to said catalytic material, wherein equal to or more than 55 atomic-%, more preferably equal to or more than 60 atomic-%, more preferably equal to or more than 65 atomic-%, more preferably equal to or more than 70 atomic-%, more preferably equal to or more than 75 atomic-%, more preferably equal to or more than 80 atomic-%, more preferably equal to or more than 85 atomic-%, of the Ni are in an oxidation state of 0.

A further preferred embodiment (12) concretizing embodiment (11) relates to said catalytic material, comprising particles of Ni.

A further preferred embodiment (13) concretizing embodiment (12) relates to said catalytic material, exhibiting a monomodal particle size distribution of the crystallites of Ni, preferably determined according to Reference Example 4.

A further preferred embodiment (14) concretizing embodiment (13) relates to said catalytic material, wherein the particle size distribution of the crystallites of Ni displays a maximum in the range of from 2 to 20 nm, more preferably in the range of from 3 to 15 nm, more preferably in the range of from 4 to 11 nm, more preferably in the range of from 4.5 to 10.0 nm, more preferably in the range of from 5.0 to 9.0 nm, preferably determined according to Reference Example 4.

A further preferred embodiment (15) concretizing any one of embodiments (11) to (14) relates to said catalytic material, wherein the Ni has a metallic surface area in the range of from 40 to 100 $m^2$/g, more preferably in the range of from 50 to 90 $m^2$/g, more preferably in the range of from 70 to 80 $m^2$/g, preferably determined according to Reference Example 3.

A further preferred embodiment (16) concretizing any one of embodiments (1) to (15) relates to said catalytic material, exhibiting a total pore volume in the range of from 0.1 to 1.00 $cm^3$/g, preferably in the range of from 0.20 to 0.50 $cm^3$/g, more preferably in the range of from 0.25 to 0.45 $cm^3$/g, preferably determined according to Reference Example 2.

A further preferred embodiment (17) concretizing any one of embodiments (1) to (16) relates to said catalytic material, comprising from 0 to 2 weight-%, more preferably from 0.001 to 1 weight-%, more preferably from 0.01 to 0.5 weight-%, of Hf, calculated as $HfO_2$, wherein the catalytic material is more preferably essentially free of Hf, wherein the catalytic material does more preferably not comprise Hf.

A further preferred embodiment (18) concretizing any one of embodiments (1) to (17) relates to said catalytic material, comprising from 0 to 1 weight-%, more preferably from 0.001 to 0.5 weight-%, more preferably from 0.01 to 0.1 weight-%, of an alkali metal, calculated as elemental alkali metal, wherein the catalytic material is more preferably essentially free of an alkali metal, wherein the catalytic material does more preferably not comprise an alkali metal.

A further preferred embodiment (19) concretizing embodiment (18) relates to said catalytic material, wherein the alkali metal comprises one or more of Li, Na, and K, more preferably Na.

A further preferred embodiment (20) concretizing any one of embodiments (1) to (19) relates to said catalytic material, further comprising an element E selected from the group consisting of Mg, Ca, Zn, B, Fe, Cl, and a mixture of two or more thereof.

A further preferred embodiment (21) concretizing embodiment (20) relates to said catalytic material, comprising from 0 to 1 weight-%, more preferably from 0.001 to 0.5 weight-%, more preferably from 0.01 to 0.1 weight-%, of the element E, calculated as elemental E, based on the total weight of the catalytic material.

A further preferred embodiment (22) concretizing any one of embodiments (1) to (21) relates to said catalytic material, wherein from 90 to 100 weight-%, more preferably from 95 to 100 weight-%, more preferably from 99 to 100 weight-%, more preferably from 99.9 to 100 weight-%, of the catalytic material consist of Ni, Si, Zr, O and H.

A further preferred embodiment (23) concretizing any one of embodiments (1) to (22) relates to said catalytic material, comprising from 0 to 3 weight-%, more preferably from 0.001 to 1 weight-%, more preferably from 0.01 to 0.5 weight-%, of C, calculated as elemental C, wherein the catalytic material more preferably is essentially free of C, wherein the catalytic material more preferably does not comprise C.

A further preferred embodiment (24) concretizing any one of embodiments (1) to (23) relates to said catalytic material, being in the form of a molding.

A further preferred embodiment (25) concretizing embodiment (24) relates to said catalytic material, further comprising an auxiliary agent selected from the group consisting of graphite, a polysaccharide, a sugar alcohol, a synthetic polymer, and a mixture of two or more thereof, more preferably selected from the group consisting of graphite, a sugar alcohol, a synthetic polymer, cellulose, a modified cellulose, a starch, and a mixture of two or more thererof, more preferably selected from the group consisting of graphite, a sugar alcohol, a synthetic polymer, a microcrystalline cellulose, a cellulose ether, and a mixture of two or more thereof, more preferably selected from the group consisting of graphite, sorbitol, mannitol, polyethylene glycol (PEG), polyvinylpyrrolidone (PVP), hydroxypropyl cellulose (HPC), hydroxypropyl methylcellulose (HPMC), and a mixture of two or more thereof, wherein the auxiliary agent more preferably comprises, more preferably consist of, graphite.

A further preferred embodiment (26) concretizing embodiment (24) or (25) relates to said catalytic material, wherein the catalytic material comprises the auxiliary agent in an amount in the range of from 1.0 to 5.0 weight-%, more preferably in the range of from 2.0 to 4.0 weight-%, more preferably in the range of from 2.5 to 3.5 weight-%, based on the total weight of the catalytic material.

A further preferred embodiment (27) concretizing any one of embodiments (24) to (26) relates to said catalytic material, being in the form of a tablet, more preferably a tablet having a cylindrical shape or an ellipsoidal shape.

A further preferred embodiment (28) concretizing embodiment (27) relates to said catalytic material, wherein the tablet has a cylindrical shape, wherein the diameter is in the range of from 5 to 15 mm, more preferably in the range of from 8 to 12 mm, more preferably in the range of from 9 to 11 mm, and wherein the height is preferably in the range of from 3 to 13 mm, preferably in the range of from 6 to 10 mm, more preferably in the range of from 7 to 9 mm.

A further preferred embodiment (29) concretizing embodiment (27) or (28) relates to said catalytic material, wherein the tablet has a cylindrical shape, and wherein the tablet has a side crushing strength in the range of from 70 to 130 N, more preferably in the range of from 80 to 120 N, preferably determined according to Reference Example 5.

An embodiment (30) of the present invention relates to a process for the preparation of a catalytic material, preferably of a catalytic material according to any one of embodiments (1) to (29), more preferably of the catalytic material of any one of embodiments (1) to (23), said process comprising

(a) providing a first aqueous solution comprising a source of Si, a second aqueous solution comprising a source of Ni, a third aqueous solution comprising a precipitation agent, and a fourth aqueous solution comprising Zr;
(b) mixing the first aqueous solution, the second aqueous solution, the third aqueous solution, and the fourth aqueous solution;
(c) heating of the mixture obtained in (b) to a temperature in the range of from 50 to 90 °C, to obtain a precursor of the catalytic material;
(d) calcining of the precursor of the catalytic material obtained in (c) in a gas atmosphere having a temperature in the range of from 300 to 600 °C.

A preferred embodiment (31) concretizing embodiment (30) relates to said process, wherein in (a) the first aqueous solution is heated to a temperature in the range of from 60 to 80 °C, more preferably in the range of from 65 to 75 °C.

A further preferred embodiment (32) concretizing embodiment (30) or (31) relates to said process, wherein (b) comprises

(b.1) adjusting the pH of the first aqueous solution to be in the range of from 5 to 9;

(b.2) feeding the second aqueous solution, the third aqueous solution, and the fourth aqueous solution into the solution obtained in (b.1), to obtain a reaction mixture, such that the pH of the reaction mixture is in the range of from 6 to 8, more preferably in the range of from 6.5 to 7.5, preferably in the range of from 6.9 to 7.1.

A further preferred embodiment (33) concretizing embodiment (32) relates to said process, wherein in (b.1) the pH is adjusted to be in the range of from 6.5 to 8.5, more preferably in the range of from 6.7 to 8.0, more preferably in the range of from 6.8 to 7.5, more preferably in the range of from 6.9 to 7.1.

A further preferred embodiment (34) concretizing embodiment (32) or (33) relates to said process, wherein in (b.1) the pH is adjusted by feeding a fifth aqueous solution comprising a source of Ni into the first aqueous solution, wherein the fifth aqueous solution more preferably has the same chemical composition as the second aqueous solution, wherein more preferably the fourth aqueous solution comprises a portion of the second aqueous solution.

A further preferred embodiment (35) concretizing embodiment (30) or (31) relates to said process, wherein (b) comprises

(b.1') mixing the first aqueous solution and the third aqueous solution, to obtain an alkaline water glass-containing solution;

(b.2') mixing the second aqueous solution and the fourth aqueous solution, to obtain a metal-containing aqueous solution

(b.3') mixing the alkaline water glass-containing solution obtained in (b.1') and the metal-containing aqueous solution, to obtain a reaction mixture, such that the pH of the reaction mixture is in the range of from 7 to 9, more preferably in the range of from 7.5 to 8.5, more preferably in the range of from 7.9 to 8.1.

A further preferred embodiment (36) concretizing any one of embodiments (30) to (35) relates to said process, wherein heating in (c) comprises heating of the mixture to a temperature in the range of from 55 to 90 °C, more preferably in the range of from 65 to 80 °C, more preferably in the range of from 70 to 75 °C.

A further preferred embodiment (37) concretizing any one of embodiments (30) to (36) relates to said process, wherein the source of Si in the one or more of the first aqueous solution and the third aqueous solution comprises one or more of a silicate, more preferably one or more of a metasilicate, an orthosilicate, and a pyrosilicate, more preferably a silicate salt, more preferably a silicate salt selected from the group consisting of alkali metal silicates and mixtures thereof, wherein the alkali metal is preferably selected from the group consisting of Li, Na, K, Rb, Cs, and mixtures of two or more thereof, more preferably from the group consisting of Li, Na, K, and mixtures of two or more thereof, wherein more preferably the alkali metal is Na and/or K, preferably Na, wherein the source of Si preferably comprises, more preferably consists of, a metasilicate, more preferably sodium silicate.

A further preferred embodiment (38) concretizing any one of embodiments (30) to (37) relates to said process, wherein the concentration of the source of Si, calculated as $SiO_2$, in the first aqueous solution is in the range of from 10 to 500 mmol/l, more preferably in the range of from 30 to 250 mmol/l, more preferably in the range of from 50 to 100 mmol/l.

A further preferred embodiment (39) concretizing any one of embodiments (30) to (38) relates to said process, wherein the source of Ni comprises one or more Ni salts, more preferably one or more Ni(ll) salts, wherein the anion of the one or more Ni salts is preferably selected from the group consisting of halides, carbonate, hydrogencarbonate, sulfate, hydrogensulfate, hydroxide, nitrate, phosphate, hydogenphosphate, dihydrogenphosphate, acetate, and combinations of two or more thereof,

more preferably from the group consisting of chloride, bromide, fluoride, hydrogencarbonate, hydrogensulfate, nitrate, dihydrogenphosphate, acetate, and combinations of two or more thereof,

more preferably from the group consisting of chloride, fluoride, nitrate, acetate, and combinations of two or more thereof, wherein more preferably the anion of the one or more Ni salts is chloride and/or nitrate, preferably nitrate, and wherein more preferably the source of Ni comprise Ni(ll) chloride, wherein more preferably the source of Ni is Ni(ll) chloride.

A further preferred embodiment (40) concretizing any one of embodiments (30) to (39) relates to said process, wherein the concentration of the source of Ni, calculated as NiO, in the second aqueous solution and/or in the fifth aqueous solution is in the range of from 0.1 to 4 mol/l, more preferably in the range of from 0.4 to 2.5 mol/l, more preferably in the range of from 0.7 to 1.4 mol/l.

A further preferred embodiment (41) concretizing any one of embodiments (30) to (40) relates to said process, wherein the ratio of the total volume of the first aqueous solution to the total volume of the second aqueous solution is in the range of from 1:50 to 1:2, more preferably in the range of from 1:30 to 1:5, more preferably in the range of from 1:10 to 1:6.

A further preferred embodiment (42) concretizing any one of embodiments (30) to (41) relates to said process, wherein the precipitation agent comprises one or more of an inorganic base and an organic base, more preferably an inorganic base, wherein preferably the precipitation agent is selected from the group consisting of hydroxides, carbonates, aluminates, and mixtures of two or more thereof,

more preferably from the group consisting of alkali metal hydroxides, alkali metal carbonates, alkali metal hydrogen carbonates, alkali metal aluminates, and mixtures of two or more thereof, more preferably from the group consisting of alkali metal carbonates, alkali metal hydrogen carbonates, alkali metal aluminates, and mixtures of two or more thereof, wherein the alkali metal is preferably selected from the group consisting of Li, Na, K, Rb, Cs, and mixtures of two or more thereof,

more preferably from the group consisting of Li, Na, K, and mixtures of two or more thereof, wherein more preferably the alkali metal is Na and/or K, preferably Na,

and wherein more preferably the precipitation agent comprises sodium carbonate, sodium hydrogen carbonate, and/or sodium aluminate, preferably sodium carbonate, wherein more preferably the precipitation agent is sodium carbonate and/or sodium hydrogen carbonate, preferably sodium carbonate.

A further preferred embodiment (43) concretizing any one of embodiments (30) to (42) relates to said process, wherein the concentration of the precipitation agent in the third aqueous solution is in the range of from 1.0 to 5.0 mol/l, more preferably in the range of from 1.4 to 3.0 mol/l, more preferably in the range of from 1.8 to 2.4 mol/l.

A further preferred embodiment (44) concretizing any one of embodiments (30) to (43) relates to said process, wherein the ratio of the total volume of the first aqueous solution to the total volume of the third aqueous solution is in the range of from 1:70 to 5:1, more preferably in the range of from 1:15 to 3:1, more preferably in the range of from 1:2 to 2:1.

A further preferred embodiment (45) concretizing any one of embodiments (30) to (44) relates to said process, wherein the concentration of the source of Zr, calculated as $ZrO_2$, in the fourth aqueous solution is in the range of from 10 to 750 mmol/l, more preferably in the range of from 20 to 600 mmol/l, more preferably in the range of from 30 to 300 mmol/l.

A further preferred embodiment (46) concretizing any one of embodiments (30) to (45) relates to said process, wherein the ratio of the total volume of the first aqueous solution to the total volume of the fourth aqueous solution is in the range of from 1:20 to 1:2, more preferably in the range of from 1:10 to 1:5, more preferably in the range of from 1:8 to 1:6.

A further preferred embodiment (47) concretizing any one of embodiments (30) to (46) relates to said process, wherein the gas atmosphere in (d) comprises a temperature in the range of from 400 to 500 °C, more preferably in the range of from 440 to 460 °C.

A further preferred embodiment (48) concretizing any one of embodiments (30) to (47) relates to said process, wherein the process further comprises after (c) and prior to (d) one or more of

(s) separating the precursor of the catalytic material obtained in (d) from the mixture by filtration or centrifugation, more preferably by filtration;
(t) drying the precursor of the catalytic material obtained from (d) or (s), more preferably the precursor of the catalytic material obtained in (s), in a gas atmosphere having a temperature in the range of from 100 to 140 °C, more preferably in the range of from 110 to 130 °C, more preferably in the range of from 115 to 125 °C.

A further preferred embodiment (49) concretizing any one of embodiments (30) to (48) relates to said process, wherein the gas atmosphere in one or more of (d) and (t) comprises one or more of nitrogen and oxygen, more preferably air.

A further preferred embodiment (50) concretizing any one of embodiments (30) to (49) relates to said process, the process further comprising

(e) treating the catalytic material obtained in (d) in a gas stream comprising nitrogen and hydrogen, wherein the catalytic material is heated to a temperature in the range of from 300 to 450 °C, more preferably in the range of from 360 to 430 °C;
(f) optionally treating the catalytic material obtained in (e) with a gas stream comprising oxygen and one or more of nitrogen and carbon dioxide for passivation, wherein the catalytic material is heated to a temperature in the range of from 30 to 80 °C, more preferably in the range of from 35 to 60 °C.

A further preferred embodiment (51) concretizing embodiment (50) relates to said process, wherein the gas stream in (e) comprises hydrogen in an amount in the range of from 1 to 50 volume-%, based on the total volume of the gas stream, and nitrogen in an amount in the range of from 50 to 99 volume-%, based on the total volume of the gas stream.

A further preferred embodiment (52) concretizing embodiment (50) or (51) relates to said process, wherein in (e) the content of hydrogen in the gas stream is adjusted such that in (e) the temperature of the catalytic material does not exceed 425 °C, wherein more preferably the temperature of the catalytic material does not exceed 385 °C.

A further preferred embodiment (53) concretizing any one of embodiments (50) to (52) relates to said process, wherein in (f) the content of oxygen in the gas stream is adjusted such that in (f) the temperature of the catalytic material does not exceed 80 °C, wherein more preferably the temperature of the catalytic material does not exceed 35 °C.

A further preferred embodiment (54) concretizing any one of embodiments (30) to (53) relates to said process, the process further comprising

(A) mixing the catalytic material obtained in (d), (e), or (f) and an auxiliary agent, to obtain a mixture;
(B) shaping the mixture obtained from (A), to obtain a catalytic material being in the form of a molding.

A further preferred embodiment (55) concretizing embodiments (54) relates to said process, wherein the auxiliary agent comprises one or more of graphite, a polysaccharide, a sugar alcohol and a synthetic polymer, more preferably one or more of graphite, a sugar alcohol, a synthetic polymer, cellulose, a modified cellulose and a starch, more preferably one or more of graphite, a sugar alcohol, a synthetic polymer, a microcrystalline cellulose, a cellulose ether, more preferably one or more of graphite, sorbitol, mannitol, polyethylene glycol (PEG), polyvinylpyrrolidone (PVP), hydroxypropyl cellulose (HPC) and hydroxypropyl methylcellulose (HPMC), more preferably graphite.

A further preferred embodiment (56) concretizing embodiment (54) or (55) relates to said process, wherein the mixture prepared in (A) comprises the auxiliary agent in an amount in the range of from 1.0 to 5.0 weight-%, preferably in the range of from 2.0 to 4.0 weight-%, more preferably in the range of from 2.5 to 3.5 weight-%, based on the total weight of the mixture.

A further preferred embodiment (57) concretizing any one of embodiments (54) to (56) relates to said process, wherein shaping in (B) comprises extruding or tableting the mixture obtained in (A), more preferably tableting the mixture obtained in (A).

A further preferred embodiment (58) concretizing any one of embodiments (54) to (57) relates to said process, wherein shaping in (B) comprises tableting the mixture to a tablet, more preferably to a tablet having a cylindrical shape or an ellipsoidal shape.

A further preferred embodiment (59) concretizing embodiment (58) relates to said process, wherein tableting is performed to obtain a tablet having a cylindrical shape, wherein the diameter of the tablet is in the range of from 5 to 15 mm, more preferably in the range of from 8 to 12 mm, more preferably in the range of from 9 to 11 mm, and wherein the height is preferably in the range of from 3 to 13 mm, preferably in the range of from 6 to 10 mm, more preferably in the range of from 7 to 9 mm.

A further preferred embodiment (60) concretizing any one of embodiments (54) to (59) relates to said process, the process further comprising

(C) treating the catalytic material being in the form of a molding obtained in (B) for reducing Ni in a gas stream comprising nitrogen and hydrogen, wherein the catalytic material being in the form of a molding is heated to a temperature in the range of from 325 to 425 °C, more preferably in the range of from 350 to 390 °C;
(D) optionally treating the catalytic material being in the form of a molding obtained from (C) with a gas stream comprising oxygen and one or more of nitrogen and carbon dioxide for passivation, wherein the molding is heated to a temperature in the range of from 30 to 80 °C, more preferably in the range of from 35 to 80 °C.

A further preferred embodiment (61) concretizing embodiment (60) relates to said process, wherein the gas stream in (C) comprises hydrogen in an amount in the range of from 1 to 50 volume-%, based on the total volume of the gas stream, and nitrogen in an amount in the range of from 50 to 99 volume-%, based on the total volume of the gas stream.

A further preferred embodiment (62) concretizing embodiment (60) or (61) relates to said process, wherein in (C) the content of hydrogen in the gas stream is adjusted such that in (C) the temperature of the molding does not exceed 425 °C, wherein in (C) the temperature of the molding does preferably not exceed 380 °C.

A further preferred embodiment (63) concretizing any one of embodiments (60) to (62) relates to said process, wherein in (D) the content of oxygen in the gas stream is adjusted such that in (D) the temperature of the molding does not exceed 80 °C, wherein in (D) the temperature of the molding does preferably not exceed 35 °C.

An embodiment (64) of the present invention relates to a catalytic material obtainable or obtained by the process of any one of embodiments (30) to (63), preferably by a process comprising (a), (b), (c), (s), (t), (e), and (f).

An embodiment (65) of the present invention relates to a use of the catalytic material according to any one of embodiments (1) to (29) and (64), as a catalyst or catalyst component, preferably for a hydrogenation reaction, in particular in a hydrogenation reaction of one or more of a nitro-group containing compound, a nitrile, an aromatic, and an olefin.

An embodiment (66) of the present invention relates to a continuous process for catalytic hydrogenation of a nitro group-containing compound, the process comprising

(I) providing a reactor comprising a reaction zone which comprises the catalytic material according to any one of embodiments (1) to (29) and (64);

(II) passing a reactant stream into the reaction zone obtained from (A), wherein the reactant stream passed into the reaction zone comprises a nitro group-containing compound and hydrogen; subjecting said reactant gas stream to reaction conditions in said reaction zone; and removing a product stream from said reaction zone, said product stream comprising an amine-group containing compound.

With respect to the reactor provided in (I), it is preferred that a loop reactor is used, in particular a loop Venturi reactor. Thus, it is preferred that a reactor is provided in (I) as disclosed in WO 2014/108351 A1. As an alternative, the reactor provided in (I) is in accordance with a reactor disclosed for the process for preparing amines as disclosed in WO 00/35852 A1.

A preferred embodiment (67) concretizing embodiment (66) relates to said continuous process, wherein in (I) the catalytic material is present in a fixed-bed and/or in a fluidized bed, more preferably in a fluidized bed.

A further preferred embodiment (68) concretizing embodiment (66) or (67) relates to said continuous process, wherein in (I) the reaction zone comprises a solvent system, wherein the solvent system more preferably comprises one or more of water, and an amine-group containing compound as obtained in (II), more preferably water, more preferably de-ionized water, more preferably water, which is obtained during the hydrogenation reaction (II).

A further preferred embodiment (69) concretizing any one of embodiments (66) to (68) relates to said continuous process, wherein in (II) the reaction conditions comprise a temperature in the range of from 100 to 150 °C, more preferably in the range of from 110 to 140 °C, more preferably in the range of from 120 to 140 °C.

A further preferred embodiment (70) concretizing any one of embodiments (66) to (69) relates to said continuous process, wherein in (II) the reaction conditions comprise a pressure in the range of from 10 to 150 bar(abs), more preferably in the range of from 15 to 50 bar(abs), more preferably in the range of from 20 to 30 bar(abs).

A further preferred embodiment (71) concretizing any one of embodiments (66) to (70) relates to said continuous process, wherein the nitro group containing compound comprises one or more of an aromatic nitro group-containing compound and an aliphatic nitro group-containing compound, wherein the aromatic nitro group-containing compound more preferably comprises one or more of nitrobenzene, 1,3-dinitrobenzene, 2,4-dinitrotoluene, 2,6-dinitrotoluene, 2,4,6-trinitrotoluene, 1,2-dimethyl-3-nitrobenzene, 1,2-dimethyl-4-nitrobenzene, 1,4-dimethyl-2-nitrobenzene, 1,3-dimethyl-2-nitrobenzene, 2,4-dimethyl-1 nitrobenzene, 1,3-dimethyl-5-nitrobenzene, 1-nitronaphthalene, 2-nitronaphthalene, 1,5-dinitronaphthalene und 1,8-dinitronaphthalene, 2-mononitrotoluene, 3- mononitrotoluene, 4-mononitrotoluene, 2-chloro-1,3-dinitrobenzene, 1-chloro-2,4-dinitrobenzene, o-chloronitrobenzene, m-chloronitrobenzene, p-chloronitrobenzene, 1,2-dichloro-4-nitrobenzene, 1,4-dichloro-2- nitrobenzene, 2,4-dichloro-1-nitrobenzene, 1,2-dichloro-3-nitrobenzene, 4-chloro-2-nitrotoluene, 4-chloro-3-nitrotoluene, 2-chloro-4-nitrotoluene, 2-chlor-6-nitrotoluene, o-nitroaniline, m-nitroaniline, and p-nitroaniline, preferably 2,4-dinitrotoluene or a mixture of 2,4- and 2,6-dinitrotoluene, and wherein the aliphatic nitro group-containing compound more preferably comprises one or more of tris(hydroxymethyl)nitromethane, 2-nitro-2-methyl-1,3-propanediol, 2-nitro-2-ethyl-1,3-propanediol, 2-nitro-1-butanol and 2-nitro-2-methyl-1-propanol.

A further preferred embodiment (72) concretizing any one of embodiments (66) to (71) relates to said continuous process, wherein the product stream comprises an amine group-containing compound, more preferably one or more of an aromatic amine group-containing compound and an aliphatic amine group-containing compound, wherein the aromatic amine group-containing compound preferably comprises one or more of aminobenzene, 1,3-diaminobenzene, 2,4-diaminotoluene, 2,6-diaminotoluene, 2,4,6-triaminotoluene, 1,2-dimethyl-3-aminobenzene, 1,2-dimethyl-4-aminobenzene, 1,4-dimethyl-2-aminobenzene, 1,3-dimethyl-2-aminobenzene, 2,4-dimethyl-1-aminobenzene, 1,3-dimethyl-5-aminobenzene, 1-aminonaphthalene, 2-aminonaphthalene, 1,5-diaminonaphthalene und 1,8-diaminonaphthalene, 2-aminotoluene, 3 aminotoluene, 4-aminotoluene, 2-chloro-1,3-diaminobenzene, 1-chloro-2,4-diaminobenzene, o-chloroaminobenzene, m-chloroaminobenzene, p-chloroaminobenzene, 1,2-dichloro-4-aminobenzene, 1,4-dichloro-2-aminobenzene, 2,4-dichloro-1-aminobenzene, 1,2-dichloro-3-aminobenzene, 4-chloro-2-aminotoluene, 4-chloro-3-aminotoluene, 2-chloro-4-aminotoluene, 2-chlor-6-aminotoluene, o-phenylenediamine, m-phenylenediamine, and p-phenylenediamine, preferably 2,4-diaminotoluene, and wherein the aliphatic amine group-containing compound preferably comprises one or more of tris(hydroxymethyl)aminomethane, 2-amino-2-methyl-1,3-propanediol, 2-amino-2-ethyl-1,3-propanediol, 2-amino-1-butanol and 2-amino-2-methyl-1-propanol.

EXPERIMENTAL SECTION

The present invention is further illustrated by the following examples and reference examples.

**Reference Example 1: Determination of the BET specific surface area specific surface area**

The BET specific surface area was determined via nitrogen physisorption at 77 K according to the method disclosed in DIN 66131.

**Reference Example 2: Determination of the total pore volume and of the average pore diameter**

The total pore volume and the average pore diameter were determined via intrusion mercury porosimetry according to standard ASTM D 4284-12.

**Reference Example 3: Determination of temperature programmed reduction (TPR) and metallic surface area**

The reduction behavior of a sample was determined by temperature programmed reduction using Micromeritics AutoChem 2950 HP Chemisorption Analyzer. 100 mg of a sample were used. The sample was treated with hydrogen at a temperature of 300 °C for 1 h before measuring the amount of desorbed hydrogen. The amount of desorbed hydrogen was measured over a temperature range from -68 to 752 °C. The Ni metallic surface area was calculated from the total amount of desorbed hydrogen. The calculation was done considering a calibration sample with a known Ni surface area.

**Reference Example 4: Determination of metal particle size distribution**

The average crystallite size of Ni was measured by XRD methods applying the Scherrer equation In particular, the crystallite size was determined using X-ray diffraction by fitting the diffracted reflection width. The software used was TOPAS 6. Instrumental contribution to reflection broadening was considered during the fitting routine using the fundamental parameter approach as described in TOPAS 6 Users Manual (Bruker AXS GmbH, Ostliche Rheinbruckenstr. 49, D-76187 Karlsruhe). This led to a reliable separation of the instrumental from the sample broadening. The sample contribution was determined using a single Lorentzian profile function that is defined by the following equation I.

$$\beta = \lambda / (L*\cos\theta) \qquad (I),$$

wherein

$\beta$ is the lorentzian full width at half maximum (FWHM),
A is the X-ray wavelength,
L is the crystallite size, and
$\theta$ is the half the scattering angle of the peak position

The entire diffraction pattern was used to model the crystallite size. Data was collected on a Bruker D8 Advance diffractometer using Cu-radiation. It was measured in Bragg-Brentano geometry from 2°-70° ($2\theta$), using a step size of 0.02°($2\theta$).

**Reference Example 5: Determination of side crushing strength**

The side crushing strength was determined with a Universal Hardness Testing Machine Zwick cLine Z010 (item no. 1006326) according to ASTM D 4179 using a crosshead speed of 14 mm/min and a cylindric indenter tool with 12,2 mm diameter.

**Reference Example 6: Determination of pH value**

The pH value was determined using a pH Meter F20 from Mettler Toledo according to the respective operating instructions of 10/2015.

**Reference Example 7: Determination of particle size**

The particle size was determined with an apparatus of Retsch Typ AS 200 control using a set of sieves according to DIN/ISO 3310-1.

**Example 1: Preparation of a catalytic material comprising Ni supported on $ZrO_2/SiO_2$**

500 g deionized water are filled in a vessel. 15 g of a water glass-containing solution (containing 2.3 g of Si calculated as $SiO_2$) are added thereto under stirring. Separately, a metal-containing solution was prepared under stirring by providing 400 g of a nickel nitrate-solution (containing 56 g of Ni calculated as NiO), adding 100 g of a zirconyl nitrate-solution (containing 10 g of Zr calculated as $ZrO_2$) and 500 g of de-ionized water thereto. Further, a sodium carbonate-solution was prepared separately by dissolving 200 g of sodium carbonate in 1000 g de-ionized water.

The water glass-containing solution was heated in the vessel to a temperature of 70 °C. Then, the metal-containing solution was slowly introduced. When a pH of 7 was reached, introduction of the sodium carbonate-solution was started such that the pH of the reaction mixture in the vessel remained constant at a value of 7.0. After about 1 hour, addition of the metal-containing solution and of the sodium carbonate-solution was complete and the resulting mixture was stirred at 70 °C for one hour. Then, the resulting suspension was cooled to room temperature, filtered and the resulting solids washed with de-ionized water until the conductivity of the washing water was less than 100 μS.

The resulting solids were dried overnight in air at a temperature of 120 °C and then calcined at 450 °C for two hours in air to obtain a catalytic material comprising Ni in oxidic form supported on an oxidic support comprising Zr in oxidic form and Si in oxidic form.

A sample of the calcined catalytic material was further calcined at 900 °C. The resulting catalytic material had a NiO content of 81.7 weight-% (corresponding to 64.3 weight-% Ni), a $ZrO_2$ content of 13.7 weight-%, a $SiO_2$ content of 3.1 weight-%, a $HfO_2$ content of 0.3 weight-%, and a $Na_2O$ content of 0.2 weight.-%. Thus, the resulting catalytic material exhibited a Ni:Zr atomic ratio of 9.9.

The rest of the calcined catalytic material was milled and subsequently mixed with 3 weight-% of graphite. The resulting powder was shaped into tablets having a geometry of 10 mm*8 mm. The side crushing strength of the resulting tablets was in the range of from 80 to 120 N.

The tablets were then subjected to reduction conditions by treating them in a hydrogen and nitrogen containing stream and at a maximum temperature of 380 °C. To this effect, reduction conditions were applied comprising a gas stream containing 1 volume-% of hydrogen and 99 volume-% of nitrogen and a temperature of 350 °C. Then, the hydrogen content of the gas stream was increased up to 50 volume-% of the gas stream under the provision that the temperature did not exceed 380 °C.

Then, the tablets were cooled to room temperature in a stream of nitrogen. Subsequently, the tablets were treated in a stream of nitrogen and oxygen for passivation of the surface to obtain a reduced catalytic material. The composition of the stream was adjusted such that the temperature of the tablets did not exceed 35 °C by controlling the concentration of oxygen therein. At the beginning of the reduction process, the concentration of oxygen was 0.1 volume-% and then slowly increased up to 10 volume-%.

The resulting reduced catalytic material had a reduction degree of 80 % determined according to Reference Example 3 and an average Ni particle size of 8 nm, determined according to Reference Example 4.

## Example 2: Preparation of a catalytic material comprising Ni supported on $ZrO_2$/$SiO_2$

A metal-containing solution was prepared by mixing 793 g of a nickel chloride-solution (containing 124.5 g of Ni) with a solution of 143.5 g zirconyl chloride octahydrate ($ZrOCl_2 \cdot 8\ H2O$) in 700 ml of water. Separately, an alkaline water glass-containing solution was prepared by dissolving 320 g of sodium carbonate in 100 ml of water and admixing a solution of 18 g sodium metasilicate pentahydrate ($Na_2SiO_3 \cdot 5\ H_2O$) thereto under stirring.

1500 ml of water were filled in a vessel and heated to a temperature of 75 °C. Then, the metal-containing solution and the alkaline water glass-solution were added simultaneously such that the pH of the reaction mixture in the vessel remained constant at a value of 8.0. After the addition was completed the reaction mixture was further stirred for two hours at a temperature of 75 °C under stirring.

Subsequently, the resulting suspension was cooled to room temperature, filtered and the resulting solids washed with de-ionized water until the conductivity of the washing water was less than 50 μS.

The resulting solids were dried over night at a temperature of 120 °C in air and then calcined at 450 °C for one hours in air to obtain a catalytic material comprising Ni in oxidic form supported on an oxidic support comprising Zr in oxidic form and Si in oxidic form.

A sample of the calcined solids was further calcined at 900 °C in air. The resulting catalytic material had a NiO content of 87.7 weight-% (corresponding to 68.9 weight-% Ni), a $ZrO_2$ content of 8.6 weight-%, a $SiO_2$ content of 2.0 weight-%, and a $HfO_2$ content of 0.2 weight-%. Thus, the resulting catalytic material exhibited a Ni:Zr atomic ratio of 16.8.

The rest of the calcined catalytic material was crushed. The crushed catalytic material was then subjected to reduction conditions by treating them in a hydrogen and nitrogen containing stream and at a maximum temperature of 450 °C.

Then, the crushed catalytic material was cooled to room temperature in a stream of nitrogen. Subsequently, the crushed catalytic material was treated in a stream of carbon dioxide by adjusting the carbon dioxide content of the stream such that the temperature of the catalytic material did not exceed 60 °C. Then, the catalytic material was further treated in a stream of air for passivation of the surface. The composition of the stream of air was adjusted such that the temperature of the solids did not exceed 80 °C.

The resulting reduced catalytic material had a BET specific surface area of 125 m²/g, determined according to Reference Example 1, a total pore volume of 0.30 ml/g, and an average pore diameter of 72 Å, both determined according to Reference Example 2. In addition, the resulting reduced catalytic material had a Ni metallic surface area of 75 $m^2$/g determined according to Reference Example 3 and an average Ni particle size of Ni of 6 nm, determined according to Reference Example 4.

**Example 3: Preparation of a catalytic material comprising Ni supported on $ZrO_2$/$SiO_2$**

Example 1 was repeated with the following modifications. 460 g of a nickel nitrate solution were used containing 64 g of Ni calculated as NiO. 45 g of a zirconyl nitrate solution were used containing 4.5 Zr calculated as $ZrO_2$. 10 g of a sodium water glass solution were used containing 1.6 g of Si calculated as $SiO_2$. The amount of sodium carbonate-solution was adjusted in order to hold the pH of the reaction mixture in the vessel constant at a value of 7.0.

A sample of the calcined catalytic material was further calcined at 900 °C in air. The resulting catalytic material had a NiO content of 90.7 weight-% (corresponding to 71.4 weight-% Ni), a $ZrO_2$ content of 5.8 weight-%, and a $SiO_2$ content of 2.2 weight-%. Thus, the resulting catalytic material exhibited a Ni:Zr atomic ratio of 25.9.

After subjecting the rest of the calcined catalytic material to reduction, the resulting reduced catalytic material had a reduction degree of 85 % determined according to Reference Example 3 and an average Ni particle size of 9 nm, determined according to Reference Example 4.

**Comparative Example 4: Preparation of a comparative catalytic material**

A 5 l vessel was filled with a solution of 18 g of sodium metasilicate pentahydrate ($Na_2SiO_3 \cdot 5$ in 300 ml water. Separately, a zirconyl chloride-containing solution was prepared by dissolving 143.5 g of zirconyl chloride octahydrate ($ZrOCl_2 \cdot 8$ in 750 ml of water, and a sodium carbonate solution was prepared by dissolving 375 g of sodium carbonate under stirring in 2500 ml.

The, the zirconyl chloride-containing solution was added into the vessel under stirring. After five minutes of further stirring, 793 g of a nickel chloride-containing solution (comprising 124.5 g Ni) were added thereto within further five minutes and under stirring. The resulting mixture was heated to 75 °C. Then, the sodium carbonate solution was added until a solid started to form and until a pH 8.4 was then reached. In total, 2340 g of the sodium carbonate solution of the prepared solution were added within one hour. The resulting mixture was then stirred for two hours.

Subsequently, the resulting suspension was cooled to room temperature, filtered and the resulting solids washed with de-ionized water until the conductivity of the washing water was less than 50 µS.

The resulting solids were dried over night at a temperature of 120 °C in air and then calcined at 450 °C for one hour in air to obtain a catalytic material comprising Ni in oxidic form supported on an oxidic support comprising Zr and Ni.

A sample of the calcined solids was further calcined at 900 °C in air. The resulting catalytic material had a NiO content of 76.4 weight-% (corresponding to 60.1 weight-% Ni), a $ZrO_2$ content of 20.9 weight-%, and a $SiO_2$ content of 1.8 weight-%. Thus, the resulting catalytic material exhibited a Ni:Zr atomic ratio of 6.0.

The rest of the calcined catalytic material was crushed. The crushed catalytic material was then subjected to reduction conditions by treating them in a hydrogen and nitrogen containing stream and at a maximum temperature of 425 °C.

Then, the crushed catalytic material was cooled to room temperature in a stream of nitrogen. Subsequently, the crushed catalytic material was treated in a stream of carbon dioxide by adjusting the carbon dioxide content of the stream such that the temperature of the catalytic material did not exceed 60 °C. Then, the catalytic material was further treated in a stream of air for passivation of the surface. The composition of the stream of air was adjusted such that the temperature of the solids did not exceed 80 °C.

The resulting reduced catalytic material had a BET specific surface area of 138 $m^2$/g, determined according to Reference Example 1, a total pore volume of 0.42 ml/g, and an average pore diameter of 99 Å, both determined in accordance with Reference Example 2. In addition, the resulting reduced catalytic material had a Ni metallic surface area of 68 $m^2$/g determined according to Reference Example 3 and an average crystallite size of Ni of 6 nm, determined in accordance with Reference Example 4.

**Comparative Example 5: Preparation of a comparative catalytic material**

The procedure of Example 1 was repeated with the following exceptions. 370 g of a nickel nitrate -containing (comprising 52 g Ni, calculated as NiO), 130 g of a zirconyl nitrate-containing solution (comprising 13 g Zr, calculated as $ZrO_2$), and 14 g of a water glass-containing solution (comprising 2.2 g Si, calculated as $SiO_2$) were used.

After drying and calcining, a sample of the calcined solids was further calcined at 900 °C in air. The resulting catalytic material had a NiO content of 76.7 weight-% (corresponding to 60.3 weight-% Ni), a $ZrO_2$ content of 18.6 weight-%, and a $SiO_2$ content of 3.4 weight-%. Thus, the resulting catalytic material exhibited a Ni:Zr atomic ratio of 6.8.

**Example 6: Catalytic testing**

A sample of a catalytic material was crushed under an inert gas atmosphere to particles having a particles size of smaller than 250 μm determined according to Reference Example 7. The obtained particles were suspended in water for handling in air.

As a testing unit a loop reactor (German: "Schlaufenreaktor"; for details of a loop reactor see also WO 00/35852 A1 and WO 2014/108351 A1) was used which comprised in one part an internal circulation flow having a volume of 5.6 l and in another part a tube reactor having a total volume 4.4 l. The internal circulation flow was driven by a propulsion jet (external circulation flow consisting of a product-containing solution and suspended catalytic material). The whole testing unit was thermostatized with thermal oil for dissipating heat.

2,4-dinitrotoluene was introduced close to the propulsion jet. Hydrogen was introduced into the vapour space above the internal circulation flow whereby the hydrogen feed was adjusted by pressure to ensure an adequate feed of hydrogen replacing the consumed hydrogen as quickly as possible. The formed product mixture was removed as output via a membrane which held back the catalytic material such that the amount of liquids in the reactor part comprising the internal circulation flow was kept constant. The output was analyzed periodically. A constant amount of gaseous matter was removed at the top of the vapour space such that no accumulation of gaseous by-products or impurities could occur.

The reactor was loaded with 140 +/- 2 g (calculated as material in dried form) of a catalytic material suspended in water. Further, the reaction conditions comprised a temperature of 135 +/- 2 °C, a pressure of 25 +/- 1 bar, an external circulation flow of 450 +/- 50 kg/h and a 2,4-dinitrotoluene dosing rate of 1 +/- 0.05 kg/h. The results of the tests are noted in following table 1. The reaction progress was determined after 50 h, 100 h and 150 h time on stream via the selectivity in % towards 2,4-toluenediamine, low boiling compounds and high boiling compounds (2,4-toluenediamine is abbreviated as TDA, low boiling compounds are abbreviated as LB and high boiling compounds are abbreviated as HB).

**Table 1**

| Results of catalytic conversion of 2,4-dinitrotoluene to 2,4-toluenediamine using the catalytic materials according to Examples 1-3 and Comparative Examples 4-5. | | | | | | | | | |
|---|---|---|---|---|---|---|---|---|---|
| Example | TDA sel. [%] after 50 h | TDA sel. [%] after 100 h | TDA sel. [%] after 150 h | LB sel. [%] after 50 h | LB sel. [%] after 100 h | LB sel. [%] after 150 h | HB sel. [%] after 50 h | HB sel. [%] after 100 h | HB sel. [%] after 150 h |
| 1 | 98.5 | 98.6 | 98.7 | 0.2 | 0.1 | 0.2 | 1.3 | 1.3 | 1.1 |
| 2 | 99.0 | 98.8 | - | 0.2 | 0.2 | - | 1.0 | 1.0 | - |
| 3 | 98.5 | 98.5 | 98.5 | 0.2 | 0.2 | 0.2 | 1.3 | 1.3 | 1.3 |
| Comp. 4 | 97.9 | 98.2 | 97.3 | 0.3 | 0.2 | 0.5 | 1.7 | 1.6 | 2.2 |
| Comp. 5 | 98.1 | 97.9 | - | 0.4 | 0.4 | - | 1.5 | 1.7 | - |

It can be gathered from the results that the catalytic materials according to the present invention achieve a higher TDA selectivity after 50 h as well as after 100 h time on stream. In particular, the selectivity towards TDA was about 0.5-1 % higher in comparison to the selectivity achieved by the catalytic materials according to Comparative Examples 4 and 5. Further, the selectivity towards by-products was comparatively lower for the catalytic materials according to the present invention. In particular, the selectivity towards high boiling compounds (compounds having a higher retention time compared to TDA isomers) as well as towards low boiling compounds (compounds having a lower retention time compared to TDA isomers) was lower for the catalytic materials according to the present invention compared to the catalytic materials of Comparative Examples 4 and 5.

Examples 1 and 3 also showed a higher selectivity after 150 h time on stream compared to Comparative Example 4. It can be gathered from the results for Examples 1 and 3 in comparison to Comparative Example 4 that after 150 h time on stream the catalytic material according to the present invention showed a superior longevity since the selectivity towards undesired by-products as high boiling compounds and low boiling compounds did not increase.

**Cited literature**

- WO 00/51728 A1
- WO 00/51727 A1
- WO 95/24964 A1
- EP 0335222 A1
- DE 1257753
- US 2564331
- WO 00/35852 A1
- WO 2014/108351 A1

**Claims**

1.  A catalytic material comprising Ni supported on an oxidic support comprising Zr in oxidic form and Si in oxidic form, wherein the catalytic material comprises equal to or greater than 65 weight-% of Ni, calculated as elemental Ni, wherein the catalytic material exhibits a Ni:Zr atomic ratio in the range of from 8.5 to 50.0.

2.  The catalytic material of claim 1, wherein the catalytic material comprises from 70 to 99 weight-% of Ni, calculated as elemental Ni, based on the total weight of the catalytic material.

3.  The catalytic material of claim 1 or 2, comprising from 1 to 20 weight-% of Zr, calculated as $ZrO_2$, based on the total weight of the catalytic material.

4.  The catalytic material of any one of claims 1 to 3, exhibiting a Ni:Si atomic ratio in the range of from 10:1 to 50:1.

5.  The catalytic material of any one of claims 1 to 4, comprising from 0.5 to 5.3 weight-% of Si, calculated as $SiO_2$, based on the total weight of the catalytic material.

6.  The catalytic material of any one of claims 1 to 5, wherein the catalytic material is calcined.

7.  The catalytic material of any one of claims 1 to 5, wherein equal to or more than 55 atomic-% of the Ni are in an oxidation state of 0.

8.  The catalytic material of claim 7, comprising particles of Ni.

9.  The catalytic material of claim 8, exhibiting a monomodal particle size distribution of the crystallites of Ni, determined according to Reference Example 4.

10. The catalytic material of any one of claims 1 to 9, being in the form of a molding.

11. A process for preparation of a catalytic material, the process comprising

    (a) providing a first aqueous solution comprising a source of Si, a second aqueous solution comprising a source of Ni, a third aqueous solution comprising a precipitation agent, and a fourth aqueous solution comprising Zr;
    (b) mixing the first aqueous solution, the second aqueous solution, the third aqueous solution, and the fourth aqueous solution;
    (c) heating of the mixture obtained in (b) to a temperature in the range of from 50 to 90 °C, to obtain a precursor of the catalytic material;
    (d) calcining of the precursor of the catalytic material obtained in (c) in a gas atmosphere having a temperature in the range of from 300 to 600 °C.

12. A catalytic material obtainable or obtained by the process of claim 11.

13. Use of the catalytic material according to any one of claims 1 to 10 and 12, as a catalyst or catalyst component.

14. A continuous process for catalytic hydrogenation of a nitro group-containing compound, the process comprising

    (I) providing a reactor comprising a reaction zone which comprises the catalytic material according to anyone of claims 1 to 10 and 12;

(II) passing a reactant stream into the reaction zone obtained from (A), wherein the reactant stream passed into the reaction zone comprises a nitro group-containing compound and hydrogen; subjecting said reactant gas stream to reaction conditions in said reaction zone; and removing a product stream from said reaction zone, said product stream comprising an amine-group containing compound.

15. The process of claim 14, wherein in (I) the reaction zone comprises a solvent system.

Europäisches
Patentamt

European
Patent Office

Office européen
des brevets

# EUROPEAN SEARCH REPORT

Application Number

EP 20 17 2354

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 0 335 222 A1 (HOECHST AG [DE]) 4 October 1989 (1989-10-04) * examples 1-3,3c; table 1 * | 1-15 | INV. B01J21/06 B01J21/08 B01J23/755 C07C209/32 |
| X,D | WO 00/51728 A1 (KATALEUNA GMBH CATALYSTS [DE]; BIRKE PETER [DE] ET AL.) 8 September 2000 (2000-09-08) * example 2 * | 1-15 | |
| X,D | WO 95/24964 A1 (BASF AG [DE]; POLANEK PETER [DE] ET AL.) 21 September 1995 (1995-09-21) * example 1 * | 1-15 | |
| X | EP 1 163 955 A1 (KATALEUNA GMBH CATALYSTS [DE]) 19 December 2001 (2001-12-19) * example 2 * | 1-15 | |

TECHNICAL FIELDS
SEARCHED        (IPC)

B01J
C07C

The present search report has been drawn up for all claims

| Place of search | Date of completion of the search | Examiner |
|---|---|---|
| The Hague | 23 September 2020 | Deurinck, Patricia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**EP 3 903 926 A1**

## ANNEX TO THE EUROPEAN SEARCH REPORT
## ON EUROPEAN PATENT APPLICATION NO.

EP 20 17 2354

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2020

| Patent document cited in search report | | Publication date | Patent family member(s) | | Publication date |
|---|---|---|---|---|---|
| EP 0335222 | A1 | 04-10-1989 | AT | 83172 T | 15-12-1992 |
| | | | AU | 3177689 A | 05-10-1989 |
| | | | CA | 1330983 C | 26-07-1994 |
| | | | DE | 3811038 A1 | 12-10-1989 |
| | | | EP | 0335222 A1 | 04-10-1989 |
| | | | ES | 2053841 T3 | 01-08-1994 |
| | | | JP | H029445 A | 12-01-1990 |
| | | | JP | H0634920 B2 | 11-05-1994 |
| | | | US | 4956328 A | 11-09-1990 |
| | | | ZA | 892232 B | 29-11-1989 |
| WO 0051728 | A1 | 08-09-2000 | AT | 250977 T | 15-10-2003 |
| | | | CN | 1349434 A | 15-05-2002 |
| | | | DE | 19909176 A1 | 07-09-2000 |
| | | | DK | 1161297 T3 | 09-02-2004 |
| | | | EP | 1161297 A1 | 12-12-2001 |
| | | | JP | 4763891 B2 | 31-08-2011 |
| | | | JP | 2002537974 A | 12-11-2002 |
| | | | KR | 20010102483 A | 15-11-2001 |
| | | | US | 6680280 B1 | 20-01-2004 |
| | | | WO | 0051728 A1 | 08-09-2000 |
| WO 9524964 | A1 | 21-09-1995 | BR | 9507072 A | 09-09-1997 |
| | | | CA | 2185588 A1 | 21-09-1995 |
| | | | CN | 1146164 A | 26-03-1997 |
| | | | EP | 0672452 A1 | 20-09-1995 |
| | | | ES | 2101376 T3 | 01-07-1997 |
| | | | JP | 3844493 B2 | 15-11-2006 |
| | | | JP | H09510140 A | 14-10-1997 |
| | | | KR | 970701588 A | 12-04-1997 |
| | | | TW | 294612 B | 01-01-1997 |
| | | | US | 5736484 A | 07-04-1998 |
| | | | WO | 9524964 A1 | 21-09-1995 |
| EP 1163955 | A1 | 19-12-2001 | DK | 1163955 T3 | 19-07-2004 |
| | | | EP | 1163955 A1 | 19-12-2001 |

EPO FORM P0459

For more details about this annex : see Official Journal of the European Patent Office, No. 12/82

**EP 3 903 926 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0051728 A1
- WO 0051727 A1
- WO 9524964 A1
- EP 0335222 A1
- DE 1257753
- US 2564331 A
- WO 2014108351 A1
- WO 0035852 A1